# EUROPEAN PATENT APPLICATION

(11) **EP 4 596 541 A1**
(43) Date of publication of application: **06.08.2025**
(21) Application number: 23873217.6
(22) Date of filing: 27.09.2023
(51) Int. Cl.: C07D 407/04, A61K 31/351, A61P 3/10

(54) **NOVEL CRYSTALLINE FORM OF ENAVOGLIFLOZIN, AND PREPARATION METHOD THEREFOR**

(30) Priority: 28.09.2022 KR 20220123673
(71) Applicant: Daewoong Pharmaceutical Co., Ltd., Hwaseong-si, Gyenoggi-do 18623 (KR)
(72) Inventor: YOON, Youn Jung, Yongin-si Gyeonggi-do 16899 (KR); YOON, Hee Kyoon, Cheongju-si Chungcheongbuk-do 28774 (KR); CHOI, Ji Soo, Seoul 06310 (KR); JI, Hye Young, Yongin-si Gyeonggi-do 17010 (KR); LIM, Hyun Woo, Yongin-si Gyeonggi-do 17066 (KR)
(74) Representative: Plasseraud IP
(86) International application number: PCT/KR2023/015050
(87) International publication number: WO 2024/072108

(57) **Abstract**

The present invention relates to a novel crystalline form of enavogliflozin, and a preparation method therefor. The novel crystalline form of enavogliflozin, according to the present invention, has thermodynamic stability and a hygroscopic property that are superior to those of a conventionally reported crystalline form of enavogliflozin, and thus has excellent long-term storage and pharmaceutical stability. In addition, the time to reach Cmax is shorter than that of the conventionally reported crystalline form of enavogliflozin such that drug effects can be rapidly exhibited, and thus the present invention can be effectively used.

## Description

### [Technical Field]

The present invention relates to a novel crystalline form of enavogliflozin and a method of preparing the same.

### [Background Art]

The selection of pharmaceutically acceptable salts, co-crystals and polymorphs thereof has a very important impact on the development of a manufacturing process for an active pharmaceutical ingredient and the dosage form design and formulation of a drug product.

Specifically, the salts and cocrystals of an active pharmaceutical ingredient, and polymorphs thereof affect the final stage of the manufacture of an active pharmaceutical ingredient, that is, the recrystallization yield, process speed and purity in recrystallization and purification processes. Depending on the size or shape of a crystal, the speed in the crystallization process may vary, which affects the productivity and manufacturing costs.

In addition, in pharmaceutical aspects, physicochemical properties such as hygroscopicity, stability, solubility, particle flowability, and a dissolution rate are affected by a salt, a co-crystal, or a polymorph thereof and thus become factors that determine the production process, production and storage conditions, and shelf life of a drug product.

Meanwhile, when the bioavailability of a drug is affected by the physical properties of an active pharmaceutical ingredient, more attention is required in the selection of a salt, a co-crystal, and a polymorph thereof, and therefore an optimized salt or co-crystal, and a polymorph thereof is very important in terms of technology development and approval of the drug.

A purpose of the present invention is to explore a novel polymorph of enavogliflozin, develop a crystalline form that can maximize pharmacological activity by analyzing physicochemical properties, and thus provide a crystalline form more advantageous than existing crystalline forms.

Sodium glucose cotransporter 2 (SGLT-2) is a transporter that, along with sodium glucose cotransporter 1 (SGLT-1), is responsible for excessive blood sugar reabsorption in the kidneys, with SGLT-2 playing the major role. Therefore, when an SGLT-2 inhibitor blocks the SGLT-2 transporter, the amount of blood sugar excreted in the urine increases, which ultimately lowers blood sugar levels and releases calories from the blood sugar, resulting in weight loss.

One of the drugs developed as an SGLT-2 inhibitor that can be useful as a therapeutic agent for type 2 diabetes due to such effects is enavogliflozin represented by Chemical Formula 1 below, which is disclosed in Korean Unexamined Patent Application Publication No. 2014-0022086 (Patent Document 1).

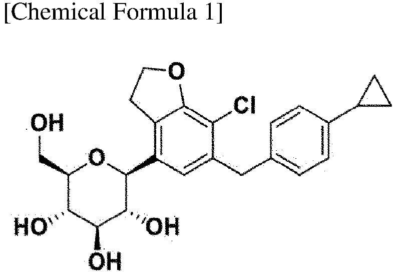

In addition, a crystalline form of enavogliflozin and a method of manufacturing a crystalline form of enavogliflozin have been disclosed in Korean Unexamined Patent Application Publication No. 2017-0142904 (Patent Document 2).

Meanwhile, the criteria for selecting a superior crystalline form are based on the most important physicochemical properties required by a drug, and depending on the purpose, such as highest thermodynamic stability, optimization for the production of an active pharmaceutical ingredient and a drug product, or improved drug solubility and dissolution rate, a different type of optimal crystalline form may be selected.

Therefore, the present inventors conducted continuous research to select a crystalline form suitable for the development of enavogliflozin products, finding the presence of crystalline form E different from the four types of crystalline forms disclosed in Korean Unexamined Patent Application Publication No. 2017-0142904 (Patent Document 2), which has no change in crystalline form, is thermodynamically more stable than existing crystalline forms, and has low hygroscopicity, thereby completing the present invention.

### [Related Art Documents]

### [Patent Documents]

Patent Document 1. Korean Unexamined Patent Application Publication No. 2014-0022086
Patent Document 2. Korean Unexamined Patent Application Publication No. 2017-0142904

### [Disclosure]

### [Technical Problem]

The present invention is directed to providing a novel crystalline form of enavogliflozin, which is thermodynamically stable and has low hygroscopicity, and a preparation method thereof. The present invention is also directed to providing a pharmaceutical composition, which includes the novel crystalline form of enavogliflozin as an active ingredient.

### [Technical Solution]

The present invention provides a novel crystalline form of enavogliflozin represented by Chemical Formula 1 below and a preparation method thereof.

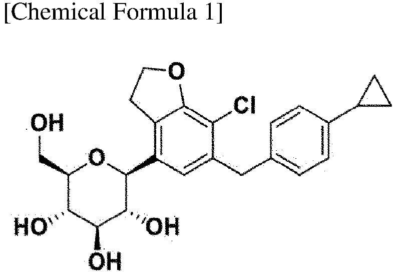

According to powder X-ray diffraction (PXRD) analysis, the novel crystalline form has a crystal structure different from the conventional four types of crystalline forms disclosed in Korean Unexamined Patent Application Publication No. 2017-0142904 (Patent Document 2).

In one embodiment of the present invention, a crystalline form of enavogliflozin (hereinafter, also referred to as crystalline form E) specified by an X-ray powder diffraction pattern having four or more, e.g., 4, 5, 6, 7, 8 or more, diffraction peaks at 2[θ] values selected from 4.93 ± 0.2, 6.12 ± 0.2, 7.43 ± 0.2, 8.89 ± 0.2, 9.74 ± 0.2, 14.79 ± 0.2, 15.79 ± 0.2, 16.11 ± 0.2, 19.79 ± 0.2, and 22.83 ± 0.2 in powder X-ray diffraction (PXRD) analysis is provided.

Particularly, the X-ray powder diffraction pattern has a diffraction peak at 2[θ] values selected from 7.43 ± 0.2, 14.79 ± 0.2, 15.79 ± 0.2, 16.11 ± 0.2, and 19.79 ± 0.2.

More specifically, the crystalline form E of enavogliflozin is specified by an X-ray powder diffraction pattern with peak positions corresponding to those listed in Table 1 below.

**[Table 1]**

| 2θ(±0.2°) | d (Å) | I/Iₒ(%) |
|---|---|---|
| 4.93 | 17.9 | 34.7 |
| 6.12 | 14.4 | 54.7 |
| 7.43 | 11.9 | 58.4 |
| 8.89 | 9.9 | 37.5 |
| 9.74 | 9.1 | 36.2 |
| 14.79 | 6.0 | 100.0 |
| 15.79 | 5.6 | 46.0 |
| 16.11 | 5.5 | 38.1 |
| 19.79 | 4.5 | 33.0 |
| 22.83 | 3.9 | 30.9 |
| 23.78 | 3.7 | 19.7 |

According to another embodiment of the present invention, the novel crystalline form E of enavogliflozin shows an endothermic peak with an endothermic onset temperature of 170.58 °C ± 3 °C and an endothermic peak temperature of 174.06 °C ± 3 °C in differential scanning calorimetry (DSC).

Another aspect of the present invention provides a method of preparing the novel crystalline form of enavogliflozin.

Although not limited thereto, the crystalline form E of enavogliflozin is prepared by a process which includes adding water to enavogliflozin, heating and stirring, and filtering and drying to obtain the crystalline form of enavogliflozin.

The method of the present invention for preparing a novel crystalline form of enavogliflozin will be described step by step in detail below.

### (a) Step of adding water to enavogliflozin

First, the method of the present invention includes adding water to solid enavogliflozin. The physicochemical form of the enavogliflozin used to prepare crystalline form E is not particularly limited. For example, the enavogliflozin used to prepare crystalline form E may be crystalline form A, crystalline form B, crystalline form C or crystalline form D of enavogliflozin, or amorphous enavogliflozin, reported to have the following X-ray diffraction spectrum, according to Experimental Example 4 in Korean Unexamined Patent Application Publication No. 2017-0142904.
Crystalline form A: the crystalline form with an X-ray diffraction (XRD) spectrum having peaks at 2[θ] values selected from 6.2° ± 0.2°, 7.2° ± 0.2°, 8.8° ± 0.2°, 17.6° ± 0.2°, 19.0° ± 0.2°, 22.5° ± 0.2°, and 25.1° ± 0.2°
Crystalline form B: the crystalline form with an X-ray diffraction (XRD) spectrum having peaks at 2[θ] values selected from 7.0° ± 0.2°, 14.9° ± 0.2°, 17.7° ± 0.2°, 18.8° ± 0.2°, 20.6° ± 0.2°, 21.8° ± 0.2°, and 23.5° ± 0.2°
Crystalline form C: the crystalline form with an X-ray diffraction (XRD) spectrum having peaks at 2[θ] values selected from 5.6° ± 0.2°, 7.3° ± 0.2°, 15.7° ± 0.2°, 17.2° ± 0.2°, 18.9° ± 0.2°, 21.2° ± 0.2°, and 21.9° ± 0.2°
Crystalline form D: the crystalline form with an X-ray diffraction (XRD) spectrum having peaks at 2[θ] values selected from 5.5° ± 0.2°, 7.2° ± 0.2°, 15.3° ± 0.2°, 17.2° ± 0.2°, 17.6° ± 0.2°, 18.9° ± 0.2°, and 21.1° ± 0.2°
Each of the crystalline forms A, B, C, and D is specified by the X-ray diffraction spectra having four or more, e.g., 4, 5, 6, 7, 8 or more, peaks at the presented 2[θ] values.

According to one embodiment of the present invention, water in step (a) may be used in a volume 6 times or more the weight of enavogliflozin, for example, 6 to 50 times, for example, 7, 8, 9, 10, 12, 15, 18, 20, 22, 25, 28, 30, 40, or 50 times, the weight of enavogliflozin.

### (b) Heating and stirring the resulting product

Next, the method of the present invention includes heating and stirring the resulting product of step (a).

According to another embodiment of the present invention, the heating in step (b) may be performed at 50 °C or more, for example, 50 to 80 °C, such as 50 to 60 °C, 60 to 70 °C, or 70 to 80 °C, but the present invention is not limited thereto.

According to still another embodiment of the present invention, the stirring in step (b) may be performed for 8 hours or more, for example, 8 to 48, such as 8 to 10, 10 to 14, 12 to 16, 16 to 20, 20 to 24, 24 to 30, 30 to 36, or 36 to 48 hours, but the present invention is not limited thereto, and the time for stirring may be appropriately adjusted depeding on factors such as the amount of water used, the heating temperature, or the like.

### (c) Drying the resulting product and obtaining crystalline form of enavogliflozin

Finally, the method of the present invention includes filtering the product of step (b), drying it to obtain a novel crystalline form E of enavogliflozin.

According to another embodiment of the present invention, the drying of step (c) may be vacuum drying, for example, at 30 to 65 °C for 8 to 24 hours. More preferably, the vacuum drying is performed at 45 to 55 °C.

By the above-described method, as an SGLT-2 inhibitor, a novel crystalline form of enavogliflozin capable of being used as a therapeutic agent for type 2 diabetes, which regulates blood sugar by inhibiting the resorption of the blood sugar in the kidneys and excreting the blood sugar through urine, may be prepared.

The present invention also provides a pharmaceutical composition comprising the novel crystalline form E of enavogliflozin as an active ingredient, and a pharmaceutically acceptable carrier.

The pharmaceutical composition may be for treating or preventing diabetes, but the present inventio is not limited thereto.

The novel crystalline form of enavogliflozin according to the present invention may be administered in a variety of oral and parenteral dosage forms in clinical administration, and is prepared using a diluent or excipient such as a filler, an extender, a binder, a wetting agent, a disintegrant, or a surfactant, which is commonly used in formulation.

### [Advantageous Effects]

Compared to the conventionally reported crystalline forms of enavogliflozin, the novel crystalline form of enavogliflozin according to the present invention exhibits superior thermodynamic stability and improved hygroscopicity, resulting in excellent long-term storage and pharmaceutical stability. Additionally, with a Tmax reduced by 1.25 times or more compared to the conventionally reported crystalline form A, it enables faster onset of action, making it highly applicable to the development of various indications and formulations.

### [Description of Drawings]

FIG. 1 shows the result of powder X-ray diffraction (PXRD) patterns of novel crystalline forms of enavogliflozin, prepared according to embodiments of the present invention.
FIG. 2 shows the result of differential scanning calorimetry (DSC) curves for the novel crystalline forms of enavogliflozin prepared according to embodiments of the present invention.

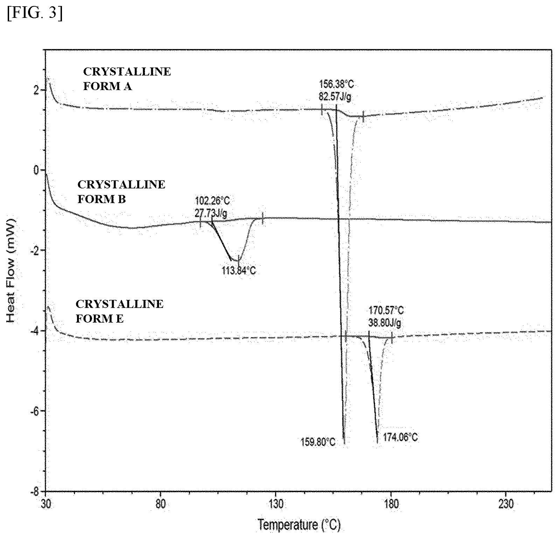

FIG. 3 shows the results of differential scanning calorimetry (DSC) for the novel crystalline forms of enavogliflozin prepared according to embodiments of the present invention and the crystalline form A and crystalline form B of enavogliflozin prepared according to embodiments of Korean Unexamined Patent Application Publication No. 2017-0142904.

### [Modes of the Invention]

Hereinafter, the advantages and features of the present invention and the methods of accomplishing the same may be clearly understood with reference to the detailed description of exemplary embodiments and the accompanying drawings. However, the present invention is not limited to the exemplary embodiments disclosed below, and may be embodied in many different forms. These exemplary embodiments are merely provided to complete the disclosure of the present invention and fully convey the scope of the present invention to those of ordinary skill in the art, and the present invention should be defined by only the accompanying claims.

### [Preparation Example 1] Preparation of crystalline form A of enavogliflozin

The crystalline form A of enavogliflozin reported according to Experimental Example 4 of Korean Unexamined Patent Application Publication No. 2017-0142904 was prepared.

Ethyl acetate (15 times the weight of crude enavogliflozin) was added to crude enavogliflozin, stirred under reflux to dissolve, and then cooled to room temperature. A suspension was formed at room temperature, and additionally stirred for 30 minutes. Isopropyl ether (15 times the weight of crude enavogliflozin) was added dropwise to the resulting mixture over 30 minutes, and further stirred at room temperature for 30 minutes. The resulting precipitate was filtered, washed with ethyl acetate (twice the weight of crude enavogliflozin) at 0 °C and dried in a vacuum oven (50 °C, 12 hrs.), thereby obtaining white crystals (yield: 88.3%).

### [Preparation Example 2] Preparation of amorphous enavogliflozin

100 mg of enavogliflozin was completely dissolved in 3 mL of tetrahydrofuran, and placed in a rotary evaporator to completely concentrate the solvent, thereby obtaining solid amorphous enavogliflozin.

### [Preparation Example 3] Preparation of novel crystalline form using crystalline form A of enavogliflozin

3 mL of water was added to 100 mg of crystalline form A of enavogliflozin to prepare a suspension, and then the suspension was stirred at 60 °C for 24 hours. The resulting product was cooled to room temperature and filtered under reduced pressure and vacuum dried at 50 °C for 12 hours, thereby obtaining a novel crystalline form of enavogliflozin with a yield of 91%.

### [Preparation Example 4] Preparation of novel crystalline form using amorphous enavogliflozin

3 mL of water was added to 100 mg of amorphous enavogliflozin to prepare a suspension, and the suspension was stirred at 60 °C for 24 hours. The resulting product was cooled to room temperature and filtered under reduced pressure and vacuum dried at 50 °C for 12 hours, thereby obtaining a novel crystalline form of enavogliflozin with a yield of 86%.

### [Experimental Example 1] Powder X-ray diffraction (PXRD)

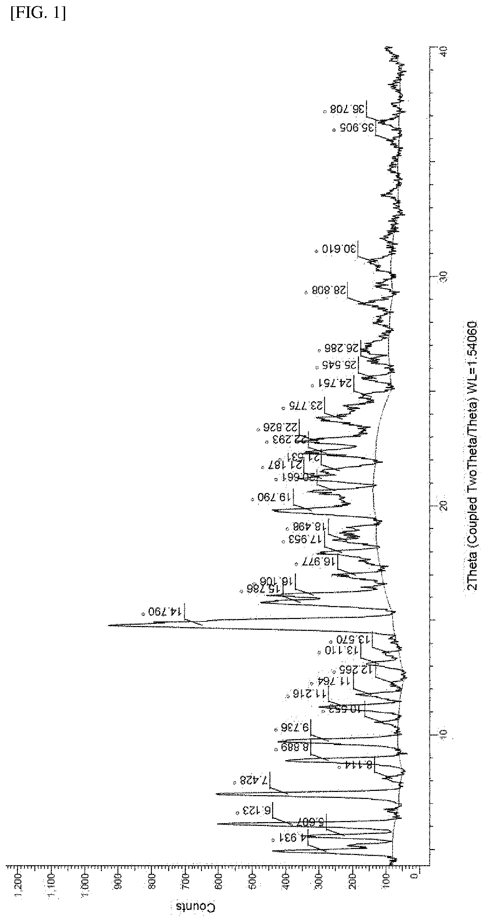

PXRD was performed using Bruker PXRD (30 kV, 10 mA, Cu target) on the crystalline forms of enavogliflozin obtained according to Preparation Examples 3 and 4. 2θ scanning was performed at 5 to 40° with 0.02° step size, obtaining the result in FIG. 1. Main peaks are summarized as shown in Table 2 below.

**[Table 2]**

| 2θ(±0.2°) | d (Å) | I/Iₒ (%) | 2θ(±0.2°) | d (Å) | I/Iₒ (%) |
|---|---|---|---|---|---|
| 4.93 | 17.9 | 34.7 | 17.95 | 4.9 | 18.5 |
| 5.61 | 15.7 | 25.5 | 18.50 | 4.8 | 15.5 |
| 6.12 | 14.4 | 54.7 | 19.79 | 4.5 | 33.0 |
| 7.43 | 11.9 | 58.4 | 20.66 | 4.3 | 19.8 |
| 8.11 | 10.9 | 4.7 | 21.19 | 4.2 | 27.0 |
| 8.89 | 9.9 | 37.5 | 21.53 | 4.1 | 18.4 |
| 9.74 | 9.1 | 36.2 | 22.29 | 4.0 | 25.9 |
| 10.55 | 8.4 | 8.2 | 22.83 | 3.9 | 30.9 |
| 11.22 | 7.9 | 27.5 | 23.78 | 3.7 | 19.7 |
| 11.76 | 7.5 | 15.4 | 24.75 | 3.6 | 8.6 |
| 12.27 | 7.2 | 5.0 | 25.55 | 3.5 | 7.6 |
| 13.11 | 5.7 | 11.8 | 26.29 | 3.4 | 5.7 |
| 13.57 | 5.5 | 4.8 | 28.81 | 3.1 | 13.6 |
| 14.79 | 6.0 | 100.0 | 30.61 | 2.9 | 7.9 |
| 15.79 | 5.6 | 46.0 | 35.91 | 2.5 | 2.8 |
| 16.11 | 5.5 | 38.1 | 36.71 | 2.4 | 7.8 |
| 16.98 | 5.2 | 13.5 | | | |

The powder X-ray diffraction (PXRD) result was different from the crystalline forms A to D of enavogliflozin previously reported in Patent Document 2, and was therefore designated as the novel crystalline form E of enavogliflozin.

### [Experimental Example 2] Differential scanning calorimetry (DSC)

DSC was performed using DSC Q20 obtained from TA Corp. at a scanning rate of 10 °C/h from 20 °C to 250 °C in nitrogen gas.

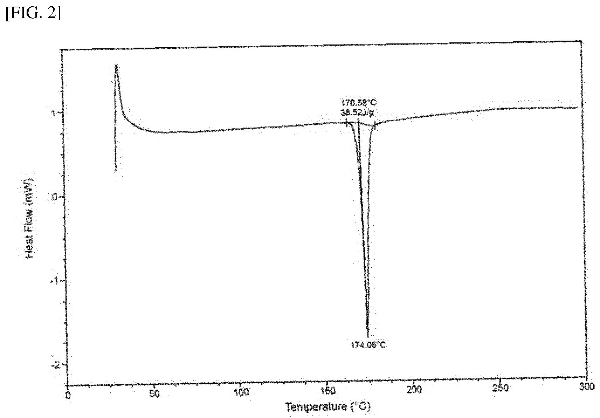

As a result, as shown in FIG. 2, the novel crystalline form E of enavogliflozin showed an endothermic peak with an endothermic onset temperature of 170.58 °C ± 3 °C and an endothermic peak temperature of 174.06 °C ± 3 °C in differential scanning calorimetry (DSC).

The melting point of the novel crystalline form E was found to be approximately 171 °C, which is the highest among the known enavogliflozin crystalline forms (see FIG. 2).

### [Experimental Example 3] Evaluation of hygroscopicity

The crystalline form A of enavogliflozin in the known Korean Unexamined Patent Application Publication No. 2017-0142904 and the enavogliflozin crystalline form E were taken in amounts of 60 mg each and placed in desiccators at relative humidity of 11%, 33%, 54%, 75%, and 93%, each for two days or more to absorb moisture, and then the weight change was measured. The results are shown in Table 3 below.

**[Table 3]**

| Relative humidity | | 11% | 33% | 54% | 75% | 93% |
|---|---|---|---|---|---|---|
| Weight change | Crystalline form E of enavogliflozin | 0% | 0.3% | 0.3% | 0.3% | 0.3% |
| | Crystalline form A of enavogliflozin | 0% | 0.5% | 0.5% | 0.5% | 0.5% |

As shown in Table 3, neither the known crystalline form A of enavogliflozin nor the crystalline form E of enavogliflozin of the present invention absorbed moisture, but it was confirmed that the novel crystalline form E of enavogliflozin is a form with more improved hygroscopicity.

### [Experimental Example 4] Evaluation of stress stability

To confirm the possibility of commercializing the novel crystalline form E of enavogliflozin of the present invention, the crystalline form A of enavogliflozin in Korean Unexamined Patent Application Publication No. 2017-0142904 was used as a control to test stress stability, and analyzed by high performance liquid chromatography (HPLC), and the results are shown in Tables 4 and 5.

Table 4 shows the results of 8-week stress stability evaluation for heat (60 °C).

**[Table 4]**

| Measurement time | | Initial | Week 2 | Week 4 | Week 8 |
|---|---|---|---|---|---|
| Purity | Crystalline form E of enavogliflozin | 99.0% | 99.2% | 99.2% | 99.5% |
| | Crystalline form A of enavogliflozin | 99.1% | 98.8% | 99.1% | 99.5% |

Table 5 shows the results of 4-week stress stability evaluation for moisture (93% RH).

**[Table 5]**

| Measurement time | | Initial | Week 2 | Week 4 |
|---|---|---|---|---|
| Purity | Crystalline form E of enavogliflozin | 99.0% | 99.3% | 99.3% |
| | Crystalline form A of enavogliflozin | 99.8% | 99.8% | 99.8% |

As shown in Tables 4 and 5, it was confirmed that the novel crystalline form E of enavogliflozin of the present invention is stably maintained for 8 weeks at 60 °C and 4 weeks at 93% RH without any effect on purity and has no change in crystalline form.

### [Experimental Example 5] Study of determining temperature range for producing crystalline form E

9 mL of water was added to 300 mg of crystalline form A of enavogliflozin and stirred at individual temperatures ranging from 40 to 80 °C for 24 hours. After filtering and vacuum drying the crystals, a crystalline form was confirmed using DSC.

**[Table 6]**

| Temperature range | Crystalline form | Yield |
|---|---|---|
| 40 to 50°C | Crystalline form A + crystalline form E mixture | 91.7% |
| 50 to 60°C | Crystalline form E | 91.5% |
| 60 to 70°C | Crystalline form E | 91.3% |
| 70 to 80°C | Crystalline form E | 94.3% |

As shown in Table 6, at 40 to 50 °C, a mixed form with crystalline form A was obtained. Therefore, it was confirmed that crystallization is preferably performed at a temperature of 50 °C or more.

### [Experimental Example 6] Study of determining crystallization time range for producing crystalline form E

9 mL of water was added to 300 mg of crystalline form A of enavogliflozin and stirred at 60 °C for 1 to 24 hours. After filtering and drying crystals, a crystalline form was confirmed using DSC.

**[Table 7]**

| Crystallization time | Crystalline form | Yield |
|---|---|---|
| 1 hour | Only crystalline form A | 87.0% |
| 4 hours | Crystalline form A + crystalline form E mixture | 90.0% |
| 8 hours | Crystalline form E | 100.0% |
| 12 hours | Crystalline form E | 92.3% |
| 24 hours | Crystalline form E | 90.7% |

As shown in Table 7, under the given conditions, it was confirmed that the crystallization time should be 8 hours or more to convert to crystalline form E. Therefore, it was confirmed that the crystallization stirring time to obtain the crystalline form E is preferably at least 8 hours or more.

### [Experimental Example 7] Study of determining water input amount for producing crystalline form E

100 mg of crystalline form A of enavogliflozin was added to 0.5 mL, 1 mL, or 2 mL of water, and stirred at 60 °C for 24 hours. After filtering and drying crystals, a crystalline form was confirmed using DSC.

**[Table 8]**

| Amount of water | Crystalline form |
|---|---|
| 0.5 mL (5 times) | Crystalline form A + crystalline form E mixture |
| 1.0 mL (10 times) | Crystalline form E |
| 2.0 mL (20 times) | Crystalline form E |

As shown in Table 8, to obtain crystalline form E, the minimum amount of water required for crystallization was checked to confirm that at least 6 times the volume of water was required compared to the weight of the crystalline form A of enavogliflozin.

### [Experimental Example 8] Pharmacokinetic study for novel crystalline form E in beagle dogs

Pharmacokinetic profiles of crystalline form A of enavogliflozin and the novel crystalline form E were to be confirmed in beagle dogs. The crystalline form A of enavogliflozin was set as a control, the novel crystalline form E was set as a comparison group, and each of the crystalline form A of enavogliflozin and the novel crystalline form E was orally administered to a male beagle dog at a dose of 1 mg/kg, and blood samples were collected before administration (0 hours), and at 0.083, 0.25, 0.5, 0.75, 1, 1.5, 2, 4, 6, 8, 12, and 24 hours (a total of 13 times). Blood drug concentrations were analyzed by LC-MS/MS using plasma separated from the collected samples, and pharmacokinetic parameters such as the maximum observed plasma concentration (Cmax), an area under the plasma concentration-time curve (AUCₗₐₛₜ and AUC_{inf}), a time-to-maximum observed plasma concentration (Tmax), and a half-life (T_{1/2}) were calculated and interpreted using the WinNonlin 5.0.1 program.

The results are summarized in Table 9 below.

**[Table 9]**

| Pharmacokinetic evaluation for crystalline form A of enavogliflozin and novel crystalline form E in beagle dogs | | |
|---|---|---|
| **Pharmacokinetic parameter** | **Crystalline form A of enavogliflozin** | **Novel crystalline form E** |
| Tmax (hr) | 4.00 | 3.20 |
| T_{1/2} (hr) | 5.32 | 5.04 |
| Cmax (ng/mL)) | 482.20 | 470.40 |
| AUCₗₐₛₜ (ng*hr/mL) | 4340.69 | 4536.00 |
| AUC_{inf} (ng*hr/mL) | 4639.38 | 4824.50 |

As shown in Table 9, it was confirmed that, compared to the crystalline form A of enavogliflozin, in the novel crystalline form E of the present invention, the time-to-maximum observed plasma concentration (Tmax) was reduced by 1.25 times or more , and the PK exposure parameters such as AUCₗₐₛₜ and AUC_{inf} also increased. Therefore, it was confirmed that the novel crystalline form E of the present invention reaches the maximum blood concentration (Tmax) earlier than the existing crystalline form A and has a faster onset of action. Accordingly, the novel crystalline form can exhibit an effect of rapidly lowering blood sugar compared to the crystalline form A of enavogliflozin.

## Claims

1. A crystalline form of enavogliflozin, which is **characterized by** an X-ray powder diffraction pattern having four or more diffraction peaks at 2[θ] values selected from 4.93 ± 0.2, 6.12 ± 0.2, 7.43± 0.2, 8.89 ± 0.2, 9.74 ± 0.2, 14.79 ± 0.2, 15.79 ± 0.2, 16.11 ± 0.2, 19.79 ± 0.2, and 22.83 ± 0.2 in powder X-ray diffraction (PXRD) analysis.

2. The crystalline form of claim 1, wherein the X-ray powder diffraction pattern has a diffraction peak at a 2[θ] value selected from 7.43 ± 0.2, 14.79 ± 0.2, 15.79 ± 0.2, 16.11 ± 0.2, and 19.79 ± 0.2.

3. The crystalline form of claim 1, which is **characterized by** an X-ray powder diffraction pattern with peak positions matching those listed in the following table:
| 2θ (±0.2°) | d (Å) | I/Iₒ(%) |
|---|---|---|
| 4.93 | 17.9 | 34.7 |
| 6.12 | 14.4 | 54.7 |
| 7.43 | 11.9 | 58.4 |
| 8.89 | 9.9 | 37.5 |
| 9.74 | 9.1 | 36.2 |
| 14.79 | 6.0 | 100.0 |
| 15.79 | 5.6 | 46.0 |
| 16.11 | 5.5 | 38.1 |
| 19.79 | 4.5 | 33.0 |
| 22.83 | 3.9 | 30.9 |
| 23.78 | 3.7 | 19.7 |

4. The crystalline form of claim 1, which shows an endothermic peak with an endothermic onset temperature of 170.58 °C ± 3 °C and an endothermic peak temperature of 174.06 °C ± 3 °C in differential scanning calorimetry (DSC).

5. A method of preparing the crystalline form of enavogliflozin of claim 1, comprising adding water to solid enavogliflozin, heating and stirring, and filtering and drying to obtain the crystalline form of enavogliflozin.

6. The method of claim 5, wherein water is added in a volume 6 times or more the weight of solid enavogliflozin.

7. The method of claim 5, wherein the heating is performed at 50 °C or more.

8. The method of claim 5, wherein the stirring is performed for 8 hours or more.

9. A pharmaceutical composition comprising the crystalline form of enavogliflozin of claim 1 as an active ingredient and a pharmaceutically acceptable carrier.

10. The composition of claim 9, which is used for treating or preventing diabetes.
